# FASCICULE DE BREVET EUROPEEN

(11) **EP 2 482 871 B1**
(45) Date de publication et mention de la délivrance du brevet: **18.03.2020**
(21) Numéro de dépôt: 10760995.0
(22) Date de dépôt: 29.09.2010
(51) Int. Cl.: A61M 5/145, A61M 5/142

(54) **PROCEDE DE CONTROLE ANTIBOLUS ET DISPOSITIF CORRESPONDANT**
KONTROLLVERFAHREN ZUM SCHUTZ VOR BOLUS UND ENTSPRECHENDE VORRICHTUNG
ANTI-BOLUS CONTROL METHOD AND CORRESPONDING DEVICE

(30) Priorité: 02.10.2009 FR 0956874
(43) Date de publication de la demande: 08.08.2012
(60) Demande divisionnaire: 19177996.6
(73) Titulaire: Fresenius Vial SAS, 38590 Brézins (FR)
(72) Inventeur: TRAVERSAZ, Philippe, 38140 Saint-Blaise du Buis (FR); ARCHAT, Damien, 38000 Grenoble (FR)
(74) Mandataire: Vièl, Frédérique
(86) Numéro de dépôt international: PCT/EP2010/064450
(87) Numéro de publication internationale: WO 2011/039250

(56) Documents cités:
- EP-A2- 0 314 880
- EP-A2- 0 354 852
- DE-C1- 4 213 172
- US-A- 5 006 112
- US-A- 5 879 360

## Description

L'invention concerne un procédé de contrôle du déplacement du poussoir d'une pompe de type pousse-seringue, la pompe comprenant un boîtier, un berceau de seringue dans lequel est placée une seringue, un poussoir mobile par rapport au boîtier et pouvant être entraîné en translation parallèlement à l'axe longitudinal de la seringue par des moyens d'entraînement, et des moyens d'embrayage pour embrayer ou débrayer le poussoir sur les moyens d'entraînement. L'invention concerne également une pompe de type pousse-seringue pour la mise en oeuvre du procédé.

Les pousse-seringues sont couramment utilisés en médecine pour administrer une solution à une vitesse relativement lente, par exemple pour la thérapie de la douleur ou en anesthésie. Ils sont en général constitués d'un boîtier muni d'un berceau destiné à recevoir une seringue contenant le liquide à perfuser et d'un poussoir qui pousse sur la tête de la seringue à une vitesse définie pour laisser s'écouler le liquide contenu dans la seringue. Le déplacement du poussoir est assuré par des moyens d'entraînement constitués d'ordinaire d'une tige filetée d'entraînement entraînée en rotation par un moteur. Un écrou solidaire du poussoir est lié à la tige filetée d'entraînement, de sorte que la rotation de celle-ci provoque la translation de l'écrou et donc du poussoir. Cet écrou est habituellement constitué de deux demi-écrous dont la fonction sera expliquée plus bas.

Lors de la mise en place d'une telle seringue, il faut tout d'abord écarter le poussoir, mettre la seringue en place dans le berceau de seringue en la bloquant, notamment dans la direction axiale, par des moyens adaptés et en ramenant le poussoir en contact avec la tête de seringue. Cette tête est constituée d'une plaque radiale disposée à l'extrémité libre du piston de la seringue. Le déplacement rapide du poussoir en direction de la tête de seringue peut se faire manuellement ou bien automatiquement. Pour le déplacement manuel du poussoir, il faut tout d'abord débrayer les moyens d'entraînement. Pour cela, les deux demi-écrous sont écartés l'un de l'autre et ainsi désolidarisés de la tige filetée d'entraînement. Dans cette position débrayée, ils ne sont plus en liaison avec la tige filetée d'entraînement et il est possible de déplacer manuellement le poussoir.

Lors de cette dernière opération, il arrive qu'il se produise un bolus involontaire (administration rapide d'une dose) au moment où le poussoir vient cogner contre la tête de seringue. Or un tel bolus peut avoir des conséquences dramatiques.

*Les documents* EP 1 329 232 A1 et EP 1 374 932 A2 décrivent un dispositif de frein qui doit empêcher le piston d'avancer lorsque le poussoir entre en contact avec la tête de seringue. La seringue est mise en place de façon traditionnelle dans le berceau de seringue, puis un dispositif de freinage, sous la forme d'une lame, vient radialement en appui sur les ailes du piston et bloque ce dernier en translation. Lorsque le poussoir est avancé, il peut taper sur la tête de seringue sans que cela ne déplace le piston. Une fois le poussoir mis en contact avec la tête de seringue, le dispositif de freinage est écarté et le moteur du pousse-seringue mis en marche. L'inconvénient d'un tel dispositif de freinage réside dans le fait qu'il peut endommager le piston ou le coincer dans le cylindre de la seringue. De plus, pour fonctionner correctement, les lames doivent être suffisamment aiguisées pour venir se planter dans le piston de la seringue, sans quoi ce dernier ne serait pas suffisamment maintenu. Cependant, si les lames sont suffisamment aiguisées, elles présentent alors un danger pour le personnel qui pourrait se blesser avec ces lames, notamment en cas de dysfonctionnement. *Le document* DE-C-4213172 *décrit également un dispositif semblable.*

L'objectif de l'invention est donc de développer un nouveau procédé et une nouvelle pompe qui permettent d'empêcher l'administration involontaire d'un bolus sans risquer pour autant d'endommager la seringue ni de blesser les utilisateurs de la pompe. Un autre objectif de l'invention est de permettre toutefois l'administration d'un bolus volontaire.

Cet objectif est atteint par le procédé de l'invention tel que défini dans la revendication 1. Le poussoir est tout d'abord désolidarisé des moyens d'entraînement, ce qui permet son déplacement manuel, et la présence ou l'absence d'un contact entre le poussoir et la tête de seringue est déterminée. Si le poussoir n'est pas en contact avec la tête de seringue, il est avancé manuellement en direction de cette dernière. Dès que le poussoir entre en contact avec la tête de seringue, le mouvement d'avance du poussoir est arrêté en actionnant des moyens de blocage du mouvement du poussoir qui jusque-là étaient désactivés. Durant toute cette étape, même lorsque le poussoir est entré en contact avec la tête de seringue, celle-ci peut être déplacée en direction de la seringue par d'autres moyens que le poussoir, par exemple à l'aide d'un doigt. Le blocage du mouvement d'avance du poussoir n'est déclenché que si au début de l'opération, le poussoir n'était pas en contact avec la tête de seringue. Contrairement à l'état de la technique, ce n'est pas le piston de la seringue qui est bloqué, mais c'est le mouvement du poussoir qui est arrêté dès qu'il y a contact entre la seringue et le poussoir. Tant que le contact n'est pas réalisé, aucun élément n'est bloqué et aucun moyen de blocage n'est activé.

Une fois que le poussoir est entré en contact avec la tête de seringue et que son mouvement d'avance a été bloqué, il est possible d'embrayer à nouveau le poussoir sur les moyens d'entraînement. Cet embrayage assure un couplage du poussoir sur le moteur d'entraînement. Le poussoir ne peut donc plus bouger tant que le moteur n'est pas actionné. Il est donc possible de débloquer le poussoir.

Il arrive qu'il soit nécessaire d'administrer un bolus volontaire. Il faut donc permettre le déplacement du poussoir même lorsque celui-ci est en contact avec la tête de seringue. Pour cela, le procédé prévoit de débrayer à nouveau le poussoir des moyens d'entraînement, puis de déplacer en translation de l'amplitude souhaitée du poussoir en contact avec la tête de la seringue en direction de la seringue et enfin d'arrêter le déplacement du poussoir et d'embrayer à nouveau le poussoir sur les moyens d'entraînement. Ces étapes ne peuvent être réalisées que si au début de la procédure de bolus volontaire, le poussoir est déjà en contact avec la tête de seringue. Il est donc possible d'administrer ce bolus volontaire directement après que le poussoir a été mis en contact avec la tête de seringue et que son mouvement a été débloqué, ou bien en cours de perfusion.

Après l'étape de mise en contact du poussoir et de la tête de seringue suivi du déblocage du mouvement du poussoir, ou après l'étape de bolus volontaire, les moyens d'entraînement peuvent être mis en marche.

Lorsque le procédé est appliqué à une pompe dont le poussoir est entraîné par une tige filetée d'entraînement au moyen de deux demi-écrous pouvant être déplacés entre une position d'embrayage dans laquelle les deux demi-écrous sont rapprochés l'un de l'autre en entourant la tige filetée d'entraînement de sorte qu'ils peuvent se déplacer sur celle-ci lorsqu'elle est mise en rotation, et une position de débrayage dans laquelle les deux demi-écrous sont écartés l'un de l'autre et désolidarisés de la tige filetée d'entraînement de sorte qu'ils ne coopèrent plus avec celle-ci, il peut arriver qu'à la fin du déplacement manuel pour approcher le poussoir de la tête de seringue ou pour appliquer un bolus volontaire, les deux demi-écrous ne soient pas alignés avec le filetage de la tige filetée d'entraînement, autrement dit, qu'ils ne tombent pas dans les creux du filet de la vis d'entraînement. Il convient donc de réaliser une séquence spécifique destinée à rattraper ce décalage. Pour cela, on a prévu après l'embrayage suivant le déplacement manuel du poussoir, soit pour le rapprocher de la tête de seringue soit pour administrer un bolus volontaire, de détecter la position relative des demi-écrous, et le cas échéant, s'ils n'ont pas atteint la position embrayée, de mettre en rotation la tige filetée d'entraînement en maîtrisant l'effort engendré par le poussoir grâce par exemple à un capteur de force tant que la position embrayée n'est pas atteinte.

L'invention concerne également une pompe de type pousse-seringue selon les termes de la revendication indépendante 7 pour la mise en oeuvre du procédé. Cette pompe comprend un boîtier, un berceau de seringue destiné à recevoir une seringue, un poussoir mobile par rapport au boîtier et pouvant être entraîné en translation parallèlement à l'axe longitudinal de la seringue par des moyens d'entraînement, des moyens d'embrayage pour embrayer ou débrayer le poussoir sur les moyens d'entraînement, la pompe étant munie de moyens pour bloquer le mouvement du poussoir en direction de la seringue lorsque celui-ci entre en contact avec la tête de seringue au cours d'un déplacement d'une position sans contact avec la tête de seringue vers une position en contact avec la tête de seringue. La pompe de l'invention se caractérise par le fait que les moyens de blocage du poussoir comprennent une tige filetée de contrôle, un écrou engagé sur la tige filetée et solidaire du poussoir, un frein pouvant agir sur la tige filetée de contrôle. Ainsi, dès que le contact entre le poussoir et la tête de seringue est constaté, le frein bloque la rotation de la tige filetée de contrôle. L'écrou, lui-même bloqué en rotation par solidarisation avec le poussoir, ne peut donc plus se déplacer sur la tige filetée de contrôle. Il en va de même du poussoir qui est solidaire de cet écrou. Le blocage du poussoir se fait donc sans intervenir sur la seringue elle-même. Le dispositif ne risque donc pas de l'endommager ou de coincer le piston de la seringue.

Dans un mode de réalisation de l'invention, le frein est constitué d'un frein électromagnétique comprenant un rotor solidaire de la tige filetée de contrôle et un stator solidaire du boîtier.

Il est conforme à l'invention de munir les *moyens de blocage, en certifier le poussoir*, d'un capteur pour détecter la présence ou l'absence de contact entre le poussoir et la tête de seringue. Si ce capteur est électrique, son signal pourra être directement utilisé pour transmettre un signal de blocage au frein électromagnétique.

Il est préférable que les moyens d'entraînement soient constitués d'un moteur et d'une tige filetée d'entraînement entraînée en rotation par le moteur. Les moyens d'embrayage peuvent alors être constitués d'un levier situé à l'extérieur du boîtier, d'un arbre-poussoir fixé au levier et de deux demi-écrous pouvant être déplacés d'une position d'embrayage dans laquelle ils entourent la tige filetée d'entraînement de sorte à pouvoir coopérer avec elle et une position de débrayage dans laquelle ils sont écartés de la tige filetée d'entraînement de sorte qu'ils ne coopèrent pas avec elle.

Il est préférable de prévoir au moins un capteur d'embrayage pour déterminer si le poussoir est embrayé ou débrayer sur les moyens d'embrayage. Ces capteurs d'embrayage peuvent être placés sur le poussoir et/ou sur les moyens d'embrayage.

Un exemple de réalisation est présenté ci-dessous à l'aide des figures qui montrent :
- Figure 1 :: une vue éclatée des principaux éléments de la pompe de type pousseseringue de l'invention sans les moyens d'entraînement du poussoir ;

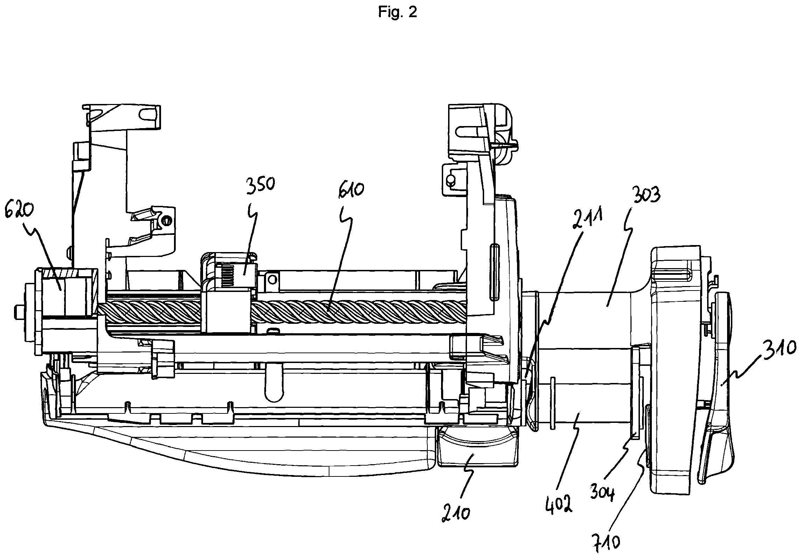

- Figure 2 :: une vue de dessus sans le capot de la pompe de la figure 1 ;
- Figure 3 :: une vue partielle de la pompe de la figure 1 montrant les moyens d'entraînement a) et b) en position embrayée, c) et d) en position débrayée, a) et c) montrant des vues générales, b) et d) des vues détaillées des demi-écrous ;

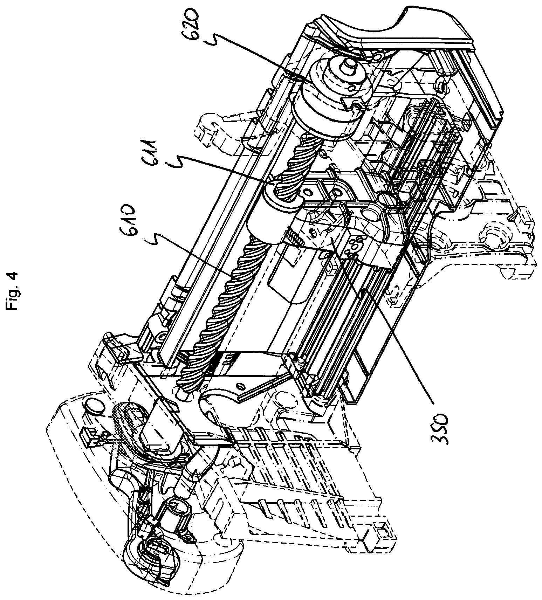

- Figure 4 :: une vue partielle de la pompe de la figure 1 montrant les moyens de contrôle ;
- Figure 5 :: a) une vue éclatée détaillée du frein électromagnétique, b1) une vue en coupe détaillée du frein électromagnétique en position désactivée et b2) en position activée, c1) une vue du détail A en position désactivée et c2) en position activée ;
- Figure 6 :: des vues des moyens d'embrayage a) en position embrayée, b) en position débrayée et c) en position d'embrayage partiel ;
- Figure 7 :: des vues du levier d'embrayage dans les positions de la figure 6 ;
- Figure 8 :: des vues de détail des demi-écrous dans les positions de la figure 6 ;
- Figure 9 :: des vues de détail du capteur opto situé au niveau du levier dans les positions de la figure 6 ;

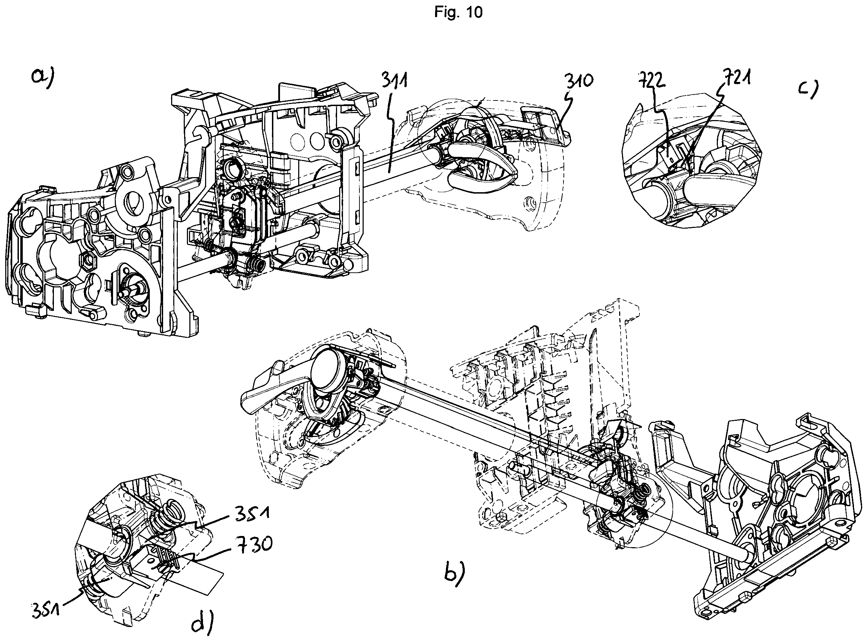

- Figure 10 :: des vues des moyens d'embrayage dans la position embrayée, a) vue générale en perspective de haut et b) en perspective de dessous, c) vue de détail du capteur opto du levier et d) vue en détail de l'interrupteur des demi-écrous ;

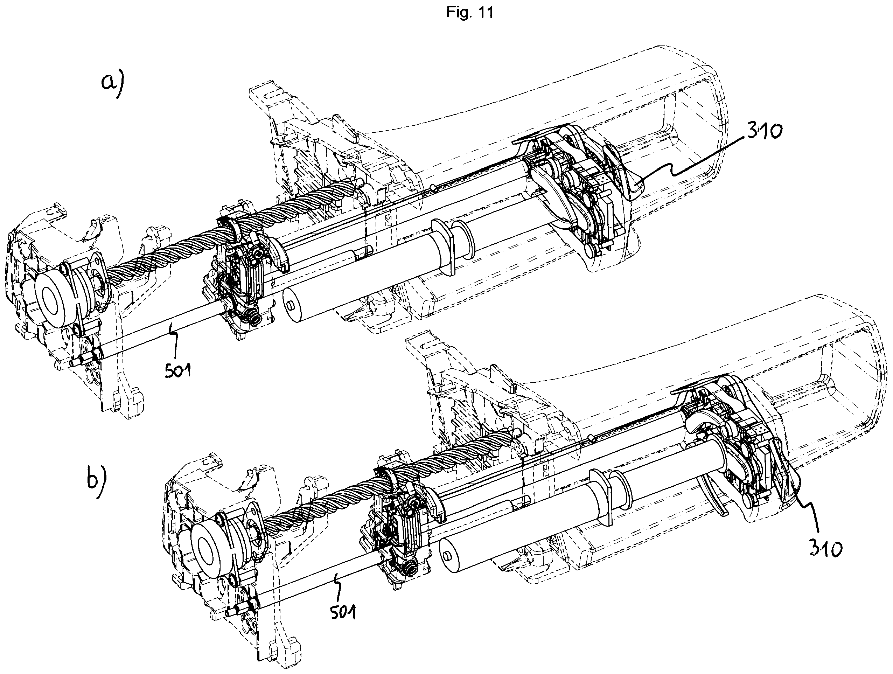

- Figure 11 :: des vues partielles générales de la pompe montrant les moyens d'entraînement, les moyens de contrôle et les moyens d'embrayage a) en position embrayée et b) en position débrayée.

Le procédé et le dispositif de l'invention sont destinés à une pompe de type pousse-seringue. Comme tout pousse-seringue, elle comprend notamment
- un boîtier (100) sur la face avant duquel se trouve
- un berceau de seringue (200) destiné à recevoir une seringue,
- un poussoir (300) destiné à pousser la tête (401) de la seringue (400) pour faire pénétrer le piston (402) de la seringue dans le cylindre (403) et ainsi expulser le contenu de la seringue et
- des moyens d'entraînement (500) pour déplacer le poussoir (300) en direction de la seringue (400) à la vitesse souhaitée.

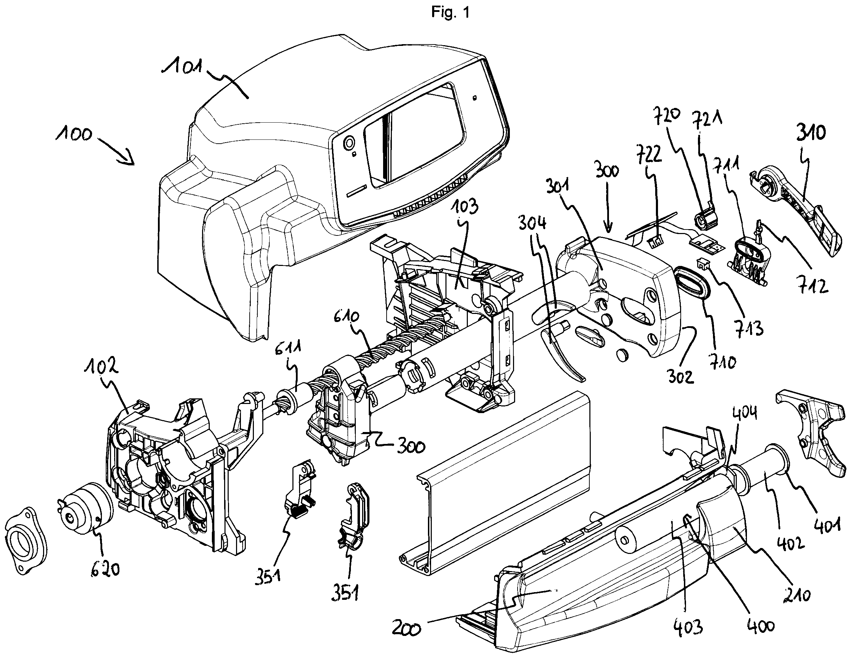

Le boîtier (100) est constitué notamment d'un capot (101), de deux flasques latéraux (102, 103), l'un (102), celui de gauche sur la figure 1 et portant le moteur étant appelé flasque moteur.

De façon connue, le berceau de seringue (200) est muni de moyens pour bloquer la seringue (400) aussi bien en direction radiale qu'en direction axiale. Ces moyens sont constitués d'une part d'un levier de maintien (210) et d'une fente (211) dans laquelle peut pénétrer l'ailette (404) du cylindre (403) de la seringue.

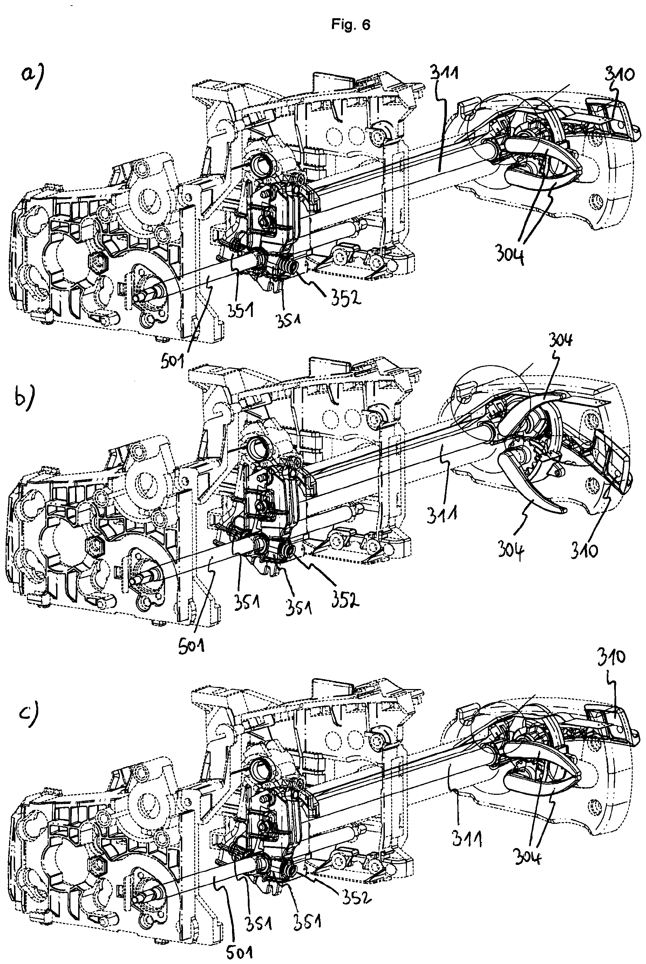

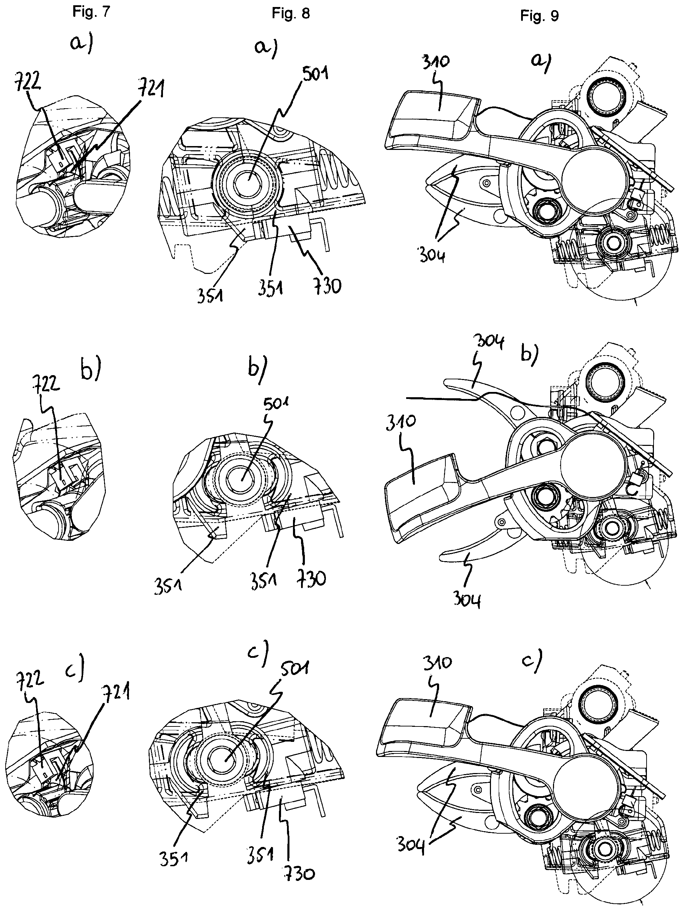

De même, le poussoir (300) est muni de moyens pour plaquer la tête de seringue (401) sur sa face avant (301), dirigée vers la seringue. Ces moyens de plaquage sont constitués de deux bras pivotants (304) qui peuvent être écartés l'un de l'autre pour laisser passer la tête de seringue (401) ou être rapprochés l'un de l'autre pour bloquer la tête de seringue sur la face avant (301) du poussoir (voir figures 6 et 9).

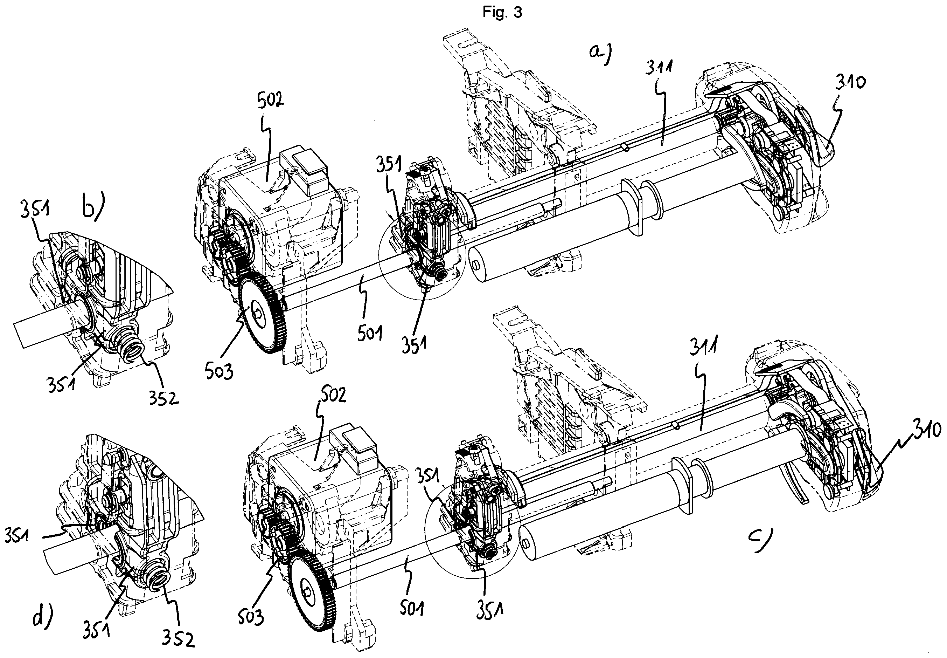

Les moyens d'entraînement (500), visibles notamment sur la figure 3, sont constitués d'une tige filetée d'entraînement (501) couplée à un moteur (502) via un réducteur (503). Ces trois organes sont placés de préférence à l'intérieur du boitier.

Le poussoir (300) est fixé au bout d'une barre de liaison (303) en forme de tube dont l'autre extrémité est fixée à un chariot (350) mobile à l'intérieur du boitier. Ce chariot (350) est couplé à la tige filetée d'entraînement (501) via un écrou. Cet écrou est lui-même constitué de deux demi-écrous (351) mobiles pouvant être déplacés d'une position dite d'embrayage dans une position dite de débrayage et vis et versa. Dans la position d'embrayage, bien visible sur la figure 8a, les demi-écrous (351) entourent la tige filetée d'entraînement (501) de sorte à coopérer avec elle pour translater le chariot (350) le long de la tige filetée d'entraînement (501) lorsque le moteur est mis en marche. Dans la position de débrayage (cf. figure 8b), au contraire, les deux demi-écrous (351) sont écartés l'un de l'autre de sorte qu'ils ne coopèrent plus avec la tige filetée d'entraînement (501). Ces deux positions sont également représentées notamment à la figure 3, la figure 3b montrant la position d'embrayage tandis que la figure 3d montre la position de débrayage. En position débrayée, il est donc possible de déplacer manuellement le chariot (350) le long de la tige filetée d'entraînement (501) sans que le moteur (502) soit mis en marche.

Le poussoir est muni de moyens de débrayage afin de le déplacer manuellement. Ces moyens de débrayage sont constitués d'un levier (310) fixé au bout d'un arbre-poussoir (311) qui est placé à l'intérieur de la barre de liaison (303). Ce levier est placé sur la face arrière (302) du poussoir, face opposée à celle destinée à pousser la tête de seringue. À l'autre extrémité de l'arbre-poussoir (311), on trouve une came (non représentée) qui coopère avec les demi-écrous (351) pour les écarter. Des ressorts (352) tendent à les rapprocher pour les mettre dans la position embrayée, tandis que la came située au bout de l'arbre-poussoir (311) les écarte lorsque le levier (310) est actionné.

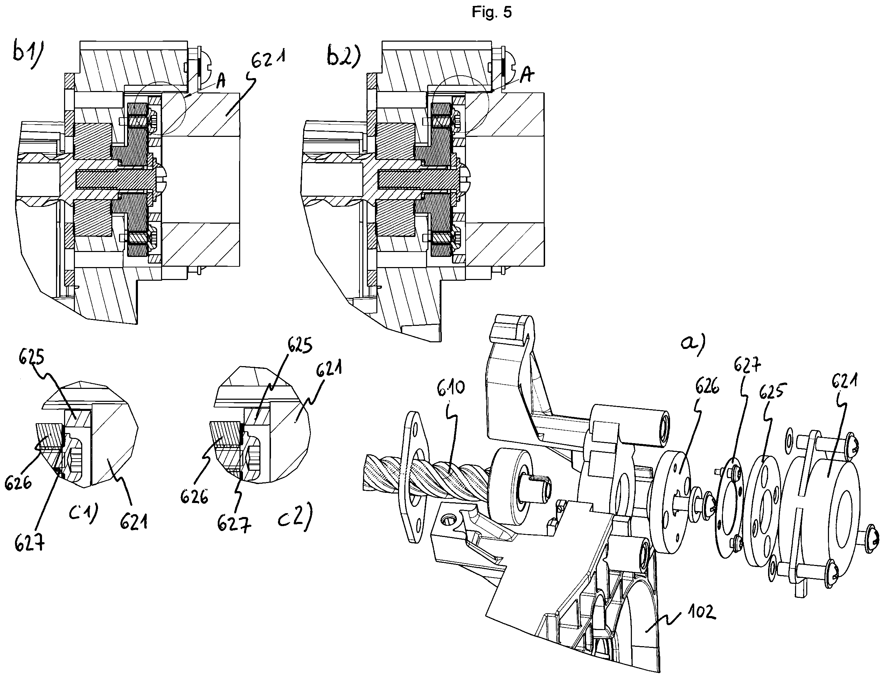

Outre ces organes communs aux pompes de l'état de la technique, le pousse-seringue de l'invention comprend des moyens de contrôle (600) qui permettent de bloquer instantanément le mouvement d'approche du poussoir (300) en direction de la tête de seringue (401). Ces moyens de contrôle sont détaillés à la figure 5. Ils sont constitués d'une part d'une tige filetée de contrôle (610) sur laquelle est monté un écrou (611) qui est lié au chariot (350) par une liaison encastrement et d'autre part d'un frein électromagnétique (620). Ce frein électromagnétique est constitué d'un stator (621) fixé au flasque moteur (102) et d'un rotor constitué d'une garniture (625) fixée avec un certain jeu axial à la tige filetée de contrôle (610) par le biais du moyeu (626). Un élément élastique (627) est interposé entre la garniture (625) et le moyeu (626). Tant que le frein électromagnétique (620) n'est pas actionné, la garniture (625) est maintenue écartée du stator (621) par l'élément élastique (627) et la tige filetée de contrôle (610) peut pivoter librement. Le chariot (350) peut donc être déplacé en translation le long de cette tige filetée de contrôle (610) soit sous l'effet des moyens d'entraînement (500), soit lors d'un déplacement manuel, ce qui provoque la rotation de la tige filetée de contrôle (610). En effet, le profil du filet de la tige filetée de contrôle (600) ainsi que de l'écrou (601) est réversible. Si par contre le frein électromagnétique est activé, les bobines contenues dans le stator (621) sont alimentées et la garniture (625) vient se coller contre la partie statique (621) du frein électromagnétique. La rotation de la tige filetée de contrôle (610) est bloquée. L'écrou de contrôle (611) étant lui-même bloqué en rotation par encastrement dans le chariot (350), c'est tout le chariot (350) et avec lui le poussoir (300) qui sont bloqués en translation.

Pour fonctionner, le dispositif de contrôle fait appel à plusieurs capteurs. Un premier capteur permet de détecter si le poussoir (300) est en contact avec la tête de seringue (401). Ce capteur de contact est constitué d'un doigt de détection (710) qui permet de faire basculer une bascule (711). Cette bascule est munie dans sa partie supérieure d'une palette (712) qui coopère avec un détecteur optique (713). Dans la position sortie du doigt de détection (710), c'est-à-dire sans contact avec la tête de seringue, la palette pénètre dans le détecteur optique (713). Si par contre le doigt de détection (710) est enfoncé, c'est-à-dire si le poussoir (300) est en contact avec la tête de seringue, alors la palette (712) sort du détecteur optique (713), ce dernier change d'état. Ce changement est utilisé pour actionner le frein électromagnétique (620). Dans une variante de réalisation, la palette se trouve hors du détecteur optique lorsque le doigt de détection est en position sortie et pénètre dans le détecteur lorsque le doigt de détection est enfoncé.

Outre ce capteur de contact, le dispositif de contrôle comprend également un capteur d'embrayage. Il est constitué d'un anneau (720) solidaire de l'arbre-poussoir (311) et il porte une palette (721) qui coopère avec un capteur optique (722). Tant que le levier (310) est en position de repos (position embrayée), la palette (721) est située dans le capteur optique (722) fournissant ainsi un signal d'embrayage. Si par contre le levier (310) est enfoncé (position débrayée), la palette (721) sort du capteur optique (722) qui fournit donc un signal de débrayage. La position de la palette (721) dans le capteur optique (722) en fonction de la position du levier (310) est bien visible sur les figures 7a/7c et 9a/9c pour la position embrayée et sur les figures 7b et 9b pour la position débrayée.

La procédure de contrôle antibolus fonctionne de la façon suivante. Le poussoir (300) se trouve en position écartée, à distance derrière la tête de seringue (401) placée de façon classique dans le berceau de seringue (200). Le doigt de détection (710) dépasse légèrement de la face avant (301) du poussoir (300) et le capteur optique de contact (713) fournit un signal d'absence de contact. Le levier étant en position de repos, le capteur optique d'embrayage (722) fournit un signal d'embrayage. Le frein électromagnétique n'est pas activé.

Le levier (310) est alors actionné en l'abaissant. Le capteur optique d'embrayage (722) fournit un signal de débrayage. L'arbre-poussoir (311) pivote, entraînant avec lui la came qui écarte les demi-écrous (351). Il est possible maintenant de déplacer le poussoir (300) en direction de la seringue. Le déplacement du poussoir (300) provoque le déplacement du chariot (350) par le biais de la barre de liaison (303). Le déplacement du chariot (350) provoque quant à lui la rotation de la tige filetée de contrôle (610) par le biais de l'écrou (611).

Dès que le poussoir (300) entre en contact avec la tête de la seringue (401), le doigt de détection (710) est enfoncé provoquant le basculement de la bascule (711) et la sortie de la palette (712) par rapport au détecteur optique de contact (713). Celui-ci émet un signal de contact qui est utilisé pour actionner le frein électromagnétique (620). Les bobines contenues dans le stator (621) sont alimentées et la garniture (625) vient se coller contre le stator, bloquant ainsi la rotation de la tige filetée de contrôle (610). Dans la variante évoquée précédemment, l'enfoncement du doigt de détection provoque la pénétration de la palette dans le détecteur optique.

Parallèlement au capteur d'embrayage, on trouve également un capteur (730) de position des demi-écrous (351). Le fait d'enfoncer le levier (310) provoque la rotation de l'arbre-poussoir (311) et l'écartement des demi-écrous (351) par le biais de la came d'écartement, tandis que le retour en position de repos du levier (310) provoque la rotation inverse de l'arbre-poussoir (311) et le retour de la came en position de repos. Sous l'effet des ressorts (352) les demi-écrous (351) devraient retourner en position fermée d'embrayage. Cependant, suite au déplacement manuel du chariot (350), il est possible que les filets des demi-écrous (351) ne soient pas en face du filet de la tige filetée d'entraînement (501). Dans ce cas, les demi-écrous (351) ne peuvent pas reprendre leur position d'embrayage bien que le levier soit en position de repos. On a donc prévu un capteur de type interrupteur (730) situé sur les demi-écrous (351). Si les demi-écrous sont écartés, comme sur les figures 8b et 8c, ce capteur de position fournit un signal de position écartée (ou de débrayage). Par contre, s'ils sont en position fermée, comme sur la figure 8a, il fournit un signal de position fermée (ou d'embrayage).

Le contrôle de l'embrayage fonctionne donc selon le tableau suivant :

**Tableau 1 : Contrôle de l'embrayage**

| Position | Signal du capteur d'embrayage (722) | Signal du capteur de position (730) |
|---|---|---|
| Position embrayée (fig. 6a, 7a, 8a, 9a) : levier en position de repos ; demi-écrous en position fermée | embrayé | fermés |
| Position débrayée (fig. 6b, 7b, 8b, 9b) : levier enfoncé ; demi-écrous en position écartée | débrayé | écartés |
| Position intermédiaire (fig. 6c, 7c, 8c, 9c) : levier en position de repos ; demi-écrous en position écartée | embrayé | écartés |

Dans le cas de la position intermédiaire, position qui risque d'être atteinte lors du passage de la position débrayée à la position embrayée suite à un déplacement manuel du poussoir, il faut exécuter une séquence spécifique afin que les demi-écrous (351) retombent dans leur logement au niveau de la tige filetée d'entraînement (501). Cette procédure consiste à mettre en rotation la tige filetée d'entraînement (501), de préférence en contrôlant l'effort engendré par la poussoir sur la seringue via un capteur de force intégré dans le poussoir (non représenté), jusqu'à ce que les demi-écrous (351) aient atteint la position embrayée. Ainsi, on s'assure que le poussoir, lorsqu'il entre en contact avec la tête de seringue, agit sur la seringue avec une force ni trop importante ni trop faible. Si le poussoir appuyait avec une force trop importante sur la tête de seringue, il provoquerait un bolus involontaire. Si au contraire il appuyait sur la tête de seringue avec une force insuffisante, il se produirait un retard dans l'administration du médicament au début de la perfusion, car le poussoir doit tout d'abord être déplacé pendant un certain laps de temps en direction de la seringue avant de provoquer le déplacement de la tête de seringue.

Grâce à ce dispositif de contrôle, il est donc possible, sans intervenir directement sur la seringue, de bloquer le déplacement du poussoir dès que celui-ci entre en contact avec la tête de seringue. Il peut cependant être souhaitable d'appliquer un bolus volontaire. Pour cela, il faut permettre le déplacement manuel du poussoir bien qu'il soit en contact avec la tête de seringue. Pour cela, le procédé prévoit que le frein électromagnétique (620) ne soit pas activé si le levier est enfoncé alors que le capteur de contact (713) transmet déjà un signal de contact. Ainsi, il est possible d'administrer un bolus volontaire soit durant la perfusion, soit directement après que le poussoir a été mis en contact avec la tête de seringue. Dans ce dernier cas, il faut, pour pouvoir déplacer le poussoir après qu'il est entré en contact avec la tête de seringue, tout d'abord relâcher le levier pour donner un signal d'embrayage avant d'enfoncer à nouveau le levier et déplacer le poussoir avec la tête de seringue vers la seringue.

Le tableau 2 résume le procédé de contrôle en indiquant la succession des différentes étapes en fonction de la situation initiale.

**Tableau 2**

| Étapes successives dans le procédé de contrôle antibolus | | |
|---|---|---|
| Situation initiale | Signaux capteurs | Conséquences |
| Poussoir écarté, levier en position de repos | 713 : pas de contact | Il n'est pas possible de déplacer manuellement le poussoir. |
| | 722 : embrayé | |
| | 730 : embrayé | |
| Enfoncement du levier | 713 : pas de contact | Il est possible de déplacer manuellement le poussoir |
| | 722 : débrayé | |
| | 730 : débrayé | |
| Déplacement du poussoir en direction de la seringue | 713 : pas de contact | |
| | 722 : débrayé | |
| | 730 : débrayé | |
| Entrée en contact du poussoir et de la tête de seringue | 713 : contact | Activation du frein électromagnétique : blocage de la translation du poussoir |
| | 722 : débrayé | |
| | 730 : débrayé | |
| Relâchement du levier | 713 : contact | Désactivation du frein électromagnétique : déblocage de la translation du poussoir. |
| | 722 : embrayé | |
| | 730 : embrayé/débrayé*) | |
| | | Il n'est pas possible de déplacer manuellement le poussoir. |
| Mise en marche du moteur | 713 : contact | |
| | 722 : embrayé | |
| | 730 : embrayé | |
| Enfoncement du levier pour administrer un bolus volontaire | 713 : contact | Pas d'activation du frein électromagnétique, car contact poussoir/seringue au début de la manoeuvre |
| | 722 : débrayé | |
| | 730 : débrayé | |
| Déplacement manuel du poussoir | 713 : contact | Administration d'un bolus volontaire |
| | 722 : débrayé | |
| | 730 : débrayé | |
| Relâchement du levier | 713 : contact | |
| | 722 : embrayé | |
| | 730 : embrayé/débrayé*) | |

| | | |
|---|---|---|
| *) en cas de non-alignement des filets des demi-écrous avec le filet de la tige filetée d'entraînement, il faut réaliser la procédure d'alignement. | | |

Le procédé de contrôle peut donc se traduire de la façon suivante :
Étape a) : Débrayage du poussoir (300) des moyens d'entraînement (500) en appuyant sur le levier (310). Cela provoque l'écartement des demi-écrous (351). Le capteur de débrayage (722) est en position débrayée, le capteur de position (730) des demi-écrous est en position écartée.
Étape b) : le dispositif détecte la présence ou l'absence de contact entre le poussoir (300) et la tête de seringue (401) en exploitant le signal de contact du capteur de contact (713). Si le capteur de contact est en position de non-contact, la procédure continue à l'étape c). Si le capteur de contact est en position contact, la procédure continue à l'étape g').
Étape c) : Si le capteur de contact (713) est en position de non-contact, le poussoir (300) est déplacé en translation en direction de la seringue jusqu'à ce qu'il entre en contact avec la tête de seringue, ce qui fait passer le capteur de contact (713) en position contact. Ce passage de la position de non-contact à la position contact provoque l'activation du frein électromagnétique (620) et ainsi le blocage de la translation du poussoir. Durant toute cette étape, le piston (402) de la seringue est libre et pourrait être déplacé en direction de la seringue ou du poussoir par d'autres moyens que les moyens d'entraînement, par exemple avec le doigt.
Étape d) : Une fois le poussoir (300) en contact avec la tête de seringue (401) et le frein électromagnétique (620) actionné, il est possible d'embrayer à nouveau le poussoir sur les moyens d'entraînement (500) en relâchant le levier (310). Les demi-écrous (351) sont rapprochés jusqu'à coopérer à nouveau avec la tige filetée d'entraînement (501). Le capteur de contact (713) reste en position contact, le capteur d'embrayage (722) repasse en position embrayée et le capteur de position (730) repasse en position rapprochée, le cas échéant en réalisant la procédure d'alignement des étapes d') et d").
Étape e) : Le poussoir (300) étant embrayé sur les moyens d'entraînement (500), il n'est plus possible de déplacer le poussoir autrement qu'avec le moteur (502). Il est donc possible de désactiver le frein électromagnétique (620) et donc de débloquer le mouvement en translation du poussoir (300). Selon les besoins, la procédure continue à l'étape i) avec la mise en marche du moteur et la perfusion, soit elle continue à l'étape f) par l'administration d'un bolus volontaire.
Étape f) : Si maintenant que le poussoir (300) est en contact avec la tête de seringue (401), autrement dit que le capteur de contact (713) est en position contact, l'opérateur veut administrer un bolus volontaire, il débraye à nouveau le poussoir des moyens d'entraînement en appuyant sur le levier (310). Une fois encore, les demi-écrous (351) sont écartés de la tige filetée d'entraînement (501). Le capteur d'embrayage (722) passe à nouveau en position débrayée et le capteur de position (730) des demi-écrous (351) passe en position écartée. Cette fois-ci, le passage du capteur d'embrayage (722) de la position embrayée à la position débrayée alors que le capteur de contact (713) est en position contact ne provoque pas l'activation du frein électromagnétique, car ce dernier capteur (713) est déjà en position de contact.
Étape g) : Le poussoir (300), et avec lui la tête de seringue (401), sont déplacés en translation en direction de la seringue (400) sur la distance souhaitée pour administrer la quantité de produit voulue. Le levier (310) reste en position appuyée pendant toute cette étape.
Étape h) : Une fois le volume souhaité administré, le déplacement du poussoir (300) est arrêté et il est embrayé sur les moyens d'entraînement (500). Pour cela, le levier (310) est relâché et les demi-écrous (351) retournent en position de coopération avec la tige filetée d'entraînement (501). Le capteur de contact (713) est en position contact, le capteur d'embrayage (722) est en position embrayée et le capteur de position (730) est en position rapprochée, le cas échéant après la réalisation des étapes h") et h"').
Étape g') : Si à l'étape b) le capteur de contact (713) est en position contact, autrement dit, si le poussoir (300) est déjà en contact avec la tête de seringue, il est possible d'administrer directement un bolus volontaire. Après l'étape b, le capteur de position (713) est en position contact, le capteur d'embrayage (722) en position débrayée et le capteur de position (730) est en position écartée. Il est donc maintenant possible de déplacer en translation le poussoir, et avec lui la tête de seringue, en direction de la seringue sur la distance souhaitée pour administrer la quantité de produit voulue. Le levier (310) reste en position appuyée pendant toute cette étape.
Étape h') : Une fois le volume souhaité administré, le déplacement du poussoir est arrêté et il est embrayé sur les moyens d'entraînement. Pour cela, le levier (310) est relâché et les demi-écrous (351) retournent en position de coopération avec la tige filetée d'entraînement (501). Le capteur de contact (713) est en position contact, le capteur d'embrayage (722) est en position embrayée et le capteur de position (730) est en position rapprochée, le cas échéant après la réalisation des étapes h") et h'").
Étape i) : Les moyens d'entraînement (500) sont mis en marche pour procéder à la perfusion. Le capteur de contact (713) est en position contact, le capteur d'embrayage (722) en position embrayée et le capteur de position (730) en position approchée. Il est donc tout à fait possible, au cours de la perfusion, d'administrer un bolus volontaire en continuant la procédure à l'étape g').
Étape d'et
Étape h") : Après chaque déplacement manuel du poussoir, la position relative des demi-écrous (351) est détectée à l'aide du capteur de position (730). Si les filets des demi-écrous (351) sont engagés dans le filet de la tige filetée d'entraînement (501), il n'y a pas de problème et la procédure peut suivre son cours avec l'étape e) ou l'étape i).
Étape d") et
Étape h"') :Si par contre les filets des demi-écrous (351) ne sont pas alignés avec le filet de la tige filetée d'entraînement (501), ils ne peuvent pas se rapprocher suffisamment pour coopérer avec celle-ci. Bien que le capteur d'embrayage (722) soit en position embrayée, le capteur de position (730) reste en position écartée (cf. figures 7c) et 8c)). Il faut alors réaliser une procédure d'alignement consistant par exemple à mettre en rotation la tige filetée d'entraînement (501), en contrôlant de préférence l'effort engendré par le poussoir sur la seringue via le capteur de force intégré au poussoir, jusqu'à ce que les filets des demi-écrous soient alignés avec le filet de la tige filetée d'entraînement. Dans cette position, les demi-écrous retombent dans leur position rapprochée, poussés par les ressorts. Le capteur de position (730) transmet alors un signal de position rapprochée et la rotation de la tige filetée d'entraînement (501) est arrêtée. La procédure normale peut donc continuer à l'étape e) ou l'étape i).

Le procédé de contrôle commence toujours par le débrayage du poussoir (étape a). En effet, quelle que soit la procédure à réaliser (préparation de la pompe par mise en contact du poussoir avec la tête de seringue, ou administration d'un bolus), il faut tout d'abord débrayer le poussoir des moyens d'entraînement pour permettre un déplacement manuel du poussoir. Dès que le poussoir est en position débrayée, le procédé détermine la position du poussoir par rapport à la tête de seringue (étape b).

Si le procédé ne détecte pas de contact avec la tête de seringue, il est possible de pousser le poussoir jusqu'au contact avec la tête de seringue (étape c). Une fois cette position atteinte, le procédé bloque le mouvement du poussoir. L'utilisateur doit à nouveau embrayer le poussoir sur les moyens d'entraînement (étape d). Le dispositif de blocage est alors désactivé (étape e). Selon les besoins, il est maintenant possible d'administrer un bolus volontaire (étapes f, g, h) ou de mettre en marche la pompe (étape i). Pour administrer un bolus volontaire, il faut à nouveau actionner le levier et débrayer le poussoir des moyens d'entraînement (étape f). Il est également possible d'écarter le poussoir de la seringue, c'est-à-dire de reculer.

Si, au contraire, le procédé détecte que le poussoir est en contact avec la tête de seringue (étape b), il est possible d'appliquer un bolus volontaire (étapes g', h'). Autrement dit, en cours de pompage, il suffit d'actionner une fois le levier pour pouvoir administrer un bolus volontaire.

Grâce au procédé et au dispositif de l'invention, il est possible non seulement d'éviter un bolus involontaire lors du rapprochement du poussoir en direction de la tête de seringue, et ce, sans intervenir sur la seringue elle-même, mais également d'administrer à tout moment un bolus volontaire, soit directement après la mise en contact du poussoir avec la tête de seringue soit au cours de la perfusion. Aucune force radiale n'étant exercée sur le piston de la seringue, il n'y a aucun risque de voir celui-ci se désaxer dans le cylindre et donc de se bloquer.

### Liste des références:

- 100: Boîtier
101
102 Flasque latéral (flasque moteur)
103 Flasque latéral

- 200: Berceau de seringue
210 Levier de maintien
211 Fente de maintien

- 300: Poussoir
301 Face avant du poussoir
302 Face arrière du poussoir
303 Barre de liaison
304 Bras pivotants
310 Levier de débrayage
311 Arbre-poussoir
350 Chariot
351 Demi-écrous
352 Ressorts

- 400: Seringue
401 Tête de seringue
402 Piston de la seringue
403 Cylindre de la seringue
404 Ailettes du cylindre de seringue

- 500: Moyens d'entraînement
- 501: Tige filetée d'entraînement
- 502: Moteur
- 503: Réducteur

- 600: Moyens de contrôle
610 Tige filetée de contrôle
611 Écrou
620 Frein électromagnétique
621 Stator
625 Garniture
626 Moyeu
627 Élément élastique
710 Doigt de détection
711 Bascule
712 Palette de la bascule
713 Capteur optique de contact
720 Anneau
721 Palette de l'anneau
722 Capteur optique d'embrayage
730 Capteur de position des demi-écrous (interrupteur)

## Revendications

1. Procédé de contrôle du déplacement du poussoir d'une pompe de type pousse-seringue, la pompe comprenant un boîtier (100), un berceau de seringue (200) dans lequel est placée une seringue (400), un poussoir (300) mobile par rapport au boîtier (100) et pouvant être entraîné en translation parallèlement à l'axe longitudinal de la seringue par des moyens d'entraînement (500), et des moyens d'embrayage (310, 311, 350, 351, 352) pour embrayer ou débrayer le poussoir (300) sur les moyens d'entraînement (500), procédé **caractérisé par** les étapes suivantes :
a) débrayage du poussoir (300) des moyens d'entraînement (500) ;
b) détection au moyen d'un capteur de contact (710, 711, 712, 713) de la présence ou l'absence de contact entre le poussoir (300) et la tête de seringue (401) ;
c) déplacement en translation du poussoir (300) en direction de la seringue (400) s'il n'y a pas de contact entre le poussoir et la tête de seringue, détection au moyen du capteur de contact de l'entrée en contact du poussoir avec la tête de seringue et actionnement immédiat de moyens de blocage du mouvement de translation du poussoir qui jusque-là étaient désactivés dès que le contact entre le poussoir et la tête de seringue est constaté.

2. Procédé selon la revendication précédente, **caractérisé par** les étapes supplémentaires suivantes réalisées après l'étape c) :
d) embrayage du poussoir (300) sur les moyens d'entraînement (500) ;
e) déblocage du mouvement de translation du poussoir (300).

3. Procédé selon la revendication précédente, **caractérisé par** les étapes supplémentaires suivantes réalisées après l'étape e) :
f) débrayage du poussoir (300) des moyens d'entraînement (500) ;
g) déplacement en translation de l'amplitude souhaitée du poussoir (300) en contact avec la tête de la seringue (401) en direction de la seringue (400) ;
h) arrêt du déplacement du poussoir (300) et embrayage du poussoir sur les moyens d'entraînement (500).

4. Procédé selon la revendication 1, **caractérisé par** les étapes supplémentaires suivantes après l'étape b) en cas de détection de la présence d'un contact entre le poussoir (300) et la tête de seringue (401) à ladite étape b) :
g') déplacement en translation de l'amplitude souhaitée du poussoir (300) en contact avec la tête de la seringue (401) en direction de la seringue ;
h') arrêt du déplacement du poussoir et embrayage du poussoir sur les moyens d'entraînement (500).

5. Procédé selon l'une des revendications 2 à 4, **caractérisé par** l'étape supplémentaire suivante réalisée après l'étape e) ou h) respectivement :
i) mise en marche des moyens d'entraînement (500) du poussoir (300).

6. Procédé selon l'une des revendications 2 à 4, ledit procédé étant appliqué à une pompe dont le poussoir (300) est entraîné par une tige filetée d'entraînement (501) au moyen de deux demi-écrous (351) pouvant être déplacés entre une position d'embrayage dans laquelle les deux demi-écrous (351) sont rapprochés l'un de l'autre en entourant la tige filetée d'entraînement (501) de sorte qu'ils peuvent se déplacer sur celle-ci lorsqu'elle est mise en rotation, et une position de débrayage dans laquelle les deux demi-écrous (351) sont écartés l'un de l'autre et de la tige filetée d'entraînement (501) de sorte qu'ils ne coopèrent plus avec celle-ci, **caractérisé par** les étapes intermédiaires suivantes réalisées après l'embrayage des étapes d) ou h) respectivement :
d'/h") détection de la position relative des demi-écrous (351) ;
d"/h"') si les demi-écrous (351) n'ont pas atteint la position embrayée, mise en rotation de la tige filetée d'entraînement (501), de préférence en contrôlant l'effort engendré par le poussoir sur la seringue via un capteur de force intégré au poussoir, jusqu'à ce que les demi-écrous (351) aient atteint la position embrayée.

7. Pompe de type pousse-seringue comprenant un boîtier (100), un berceau de seringue (200) destiné à recevoir une seringue (400), un poussoir mobile (300) par rapport au boîtier (100) et pouvant être entraîné en translation parallèlement à l'axe longitudinal de la seringue (400) par des moyens d'entraînement (500), des moyens d'embrayage (310, 311, 350, 351, 352) pour embrayer ou débrayer le poussoir (300) sur les moyens d'entraînement (500), **caractérisée en ce que** la pompe est munie de moyens pour bloquer le mouvement du poussoir (300) en direction de la seringue (400) lorsque celui-ci entre en contact avec la tête de seringue (401) au cours d'un déplacement d'une position sans contact avec la tête de seringue (401) vers une position en contact avec la tête de seringue (401), les moyens de blocage du poussoir comprenant
- une tige filetée de contrôle (610),
- un écrou (611) engagé sur la tige filetée de contrôle (610) et solidaire du poussoir (300),
- un frein (620) agissant sur la tige filetée de contrôle (610),
- un capteur de contact (710, 711, 712, 713) pour détecter la présence ou l'absence de contact entre le poussoir (300) et la tête de seringue (401).

8. Pompe selon la revendication précédente, **caractérisée en ce que** le frein (620) est constitué d'un frein électromagnétique comprenant un rotor (625, 626, 627) solidaire de la tige filetée de contrôle (610) et un stator (621) solidaire du boîtier (100).

9. Pompe selon la revendication 7 ou 8, **caractérisée en ce que** le poussoir (300) est muni du capteur de contact (710, 711, 712, 713).

10. Pompe selon l'une des revendications 7 à 9, **caractérisée en ce que** les moyens d'entraînement (500) sont constitués d'un moteur (502) et d'une tige filetée d'entraînement (501) entraînée en rotation par le moteur (502).

11. Pompe selon la revendication précédente, **caractérisée en ce que** les moyens d'embrayage sont constitués d'un levier de débrayage (310) situé à l'extérieur du boîtier (100), d'un arbre-poussoir (311) solidaire du levier (310) et de deux demi-écrous (351) pouvant être déplacés d'une position d'embrayage dans laquelle ils entourent la tige filetée d'entraînement (501) de sorte à pouvoir coopérer avec elle et une position de débrayage dans laquelle ils sont écartés de la tige filetée d'entraînement (501) de sorte qu'ils ne coopèrent pas avec elle.

12. Pompe selon l'une des revendications 7 à 11, **caractérisée en ce qu'au** moins un capteur d'embrayage (720, 721, 722, 730) est prévu pour déterminer si le poussoir (300) est embrayé ou débrayé sur les moyens d'embrayage.

13. Pompe selon la revendication précédente, **caractérisée en ce que** le ou les capteurs d'embrayage (720, 721, 722, 730) sont placés sur le poussoir (300) et/ou sur les moyens d'embrayage (350).

## Patentansprüche

1. Verfahren zur Kontrolle des Verschiebens des Schiebers einer Pumpe vom Typ Spritzenpumpe, wobei die Pumpe Folgendes umfasst: ein Gehäuse (100), eine Spritzenmulde (200), in die eine Spritze (400) eingebracht wird, einen Schieber (300), der relativ zum Gehäuse (100) beweglich ist und durch Antriebsmittel (500) parallel zur Längsachse der Spritze translatorisch angetrieben werden kann, und Kupplungsmittel (310, 311, 350, 351, 352) zum Kuppeln oder Entkuppeln des Schiebers (300) mit bzw. von den Antriebsmitteln (500), wobei das Verfahren durch die folgenden Schritte **gekennzeichnet** ist:
a) Entkuppeln des Schiebers (300) von den Antriebsmitteln (500);
b) Erfassen, mittels eines Kontaktsensors (710, 711, 712, 713), ob Kontakt zwischen dem Schieber (300) und dem Spritzenkopf (401) besteht oder nicht;
c) translatorisches Verschieben des Schiebers (300) in Richtung der Spritze (400), wenn kein Kontakt zwischen dem Schieber und dem Spritzenkopf besteht, Erfassen, mittels des Kontaktsensors, des in Kontakt Tretens des Schiebers mit dem Spritzenkopf und unmittelbares Betätigen von Mitteln zum Blockieren der translatorischen Bewegung des Schiebers, die bis zu diesem Zeitpunkt deaktiviert waren, sobald der Kontakt zwischen dem Schieber und dem Spritzenkopf festgestellt wird.

2. Verfahren nach dem vorhergehenden Anspruch, **gekennzeichnet durch** die folgenden zusätzlichen Schritte, die nach dem Schritt c) durchgeführt werden:
d) Kuppeln des Schiebers (300) mit den Antriebsmitteln (500);
e) Freigeben der translatorischen Bewegung des Schiebers (300).

3. Verfahren nach dem vorhergehenden Anspruch, **gekennzeichnet durch** die folgenden zusätzlichen Schritte, die nach dem Schritt e) durchgeführt werden:
f) Entkuppeln des Schiebers (300) von den Antriebsmitteln (500);
g) translatorisches Verschieben des Schiebers (300), der mit dem Spritzenkopf (401) in Kontakt ist, um die gewünschte Amplitude in Richtung der Spritze (400);
h) Stoppen des Verschiebens des Schiebers (300) und Kuppeln des Schiebers mit den Antriebsmitteln (500).

4. Verfahren nach Anspruch 1, **gekennzeichnet durch** die folgenden zusätzlichen Schritte nach Schritt b), falls in dem Schritt b) erfasst wird, dass ein Kontakt zwischen dem Schieber (300) und dem Spritzenkopf (401) besteht:
g') translatorisches Verschieben des Schiebers (300), der mit dem Spritzenkopf (401) in Kontakt ist, um die gewünschte Amplitude in Richtung der Spritze;
h') Stoppen des Verschiebens des Schiebers und Kuppeln des Schiebers mit den Antriebsmitteln (500).

5. Verfahren nach einem der Ansprüche 2 bis 4, **gekennzeichnet durch** den folgenden zusätzlichen Schritt, der nach dem Schritt e) bzw. nach dem Schritt h) durchgeführt wird:
i) Inbetriebsetzen der Antriebsmittel (500) des Schiebers (300).

6. Verfahren nach einem der Ansprüche 2 bis 4, wobei das Verfahren auf eine Pumpe angewendet wird, deren Schieber (300) durch eine Antriebsgewindespindel (501) mit Hilfe von zwei Halbmuttern (351) angetrieben wird, die zwischen einer Kupplungsposition, in der die zwei Halbmuttern (351) einander angenähert sind und dabei die Antriebsgewindespindel (501) umgeben, sodass sie sich auf dieser verlagern können, wenn diese in Rotation versetzt wird, und einer Entkupplungsposition, in der die zwei Halbmuttern (351) voneinander und von der Antriebsgewindespindel (501) beabstandet sind, sodass sie mit dieser nicht mehr zusammenwirken, verschoben werden können, **gekennzeichnet durch** die folgenden Zwischenschritte, die nach dem Kuppeln der Schritte d) bzw. der Schritt h) durchgeführt werden:
d'/h") Erfassen der relativen Position der Halbmuttern (351);
d"/h'") wenn die Halbmuttern (351) die gekuppelte Position nicht erreicht haben, Versetzen der Antriebsgewindespindel (501) in Rotation, vorzugsweise bei gleichzeitiger Kontrolle der von der Schieber auf die Spritze ausgeübten Kraft durch einen in den Schieber integrierten Kraftsensor, bis die Halbmuttern (351) die gekuppelte Position erreicht haben.

7. Pumpe vom Typ Spritzenpumpe, die Folgendes umfasst: ein Gehäuse (100), eine Spritzenmulde (200) für die Aufnahme einer Spritze (400), einen Schieber (300), der relativ zum Gehäuse (100) beweglich ist und durch Antriebsmittel (500) parallel zur Längsachse der Spritze (400) translatorisch angetrieben werden kann, Kupplungsmittel (310, 311, 350, 351, 352) zum Kuppeln oder Entkuppeln des Schiebers (300) mit bzw. von den Antriebsmitteln (500), **dadurch gekennzeichnet, dass** die Pumpe Mittel aufweist, um die Bewegung des Schiebers (300) in Richtung der Spritze (400) zu blockieren, wenn dieser im Laufe einer Verschiebung von einer Position ohne Kontakt mit dem Spritzenkopf (401) in eine Position mit Kontakt mit dem Spritzenkopf (401) in Kontakt mit dem Spritzenkopf (401) kommt, wobei die Mittel zum Blockieren des Schiebers Folgendes umfassen:
- eine Kontrollgewindespindel (610),
- eine Mutter (611), die mit der Kontrollgewindespindel (610) in Eingriff und mit dem Schieber (300) verbunden ist,
- eine Bremse (620), die auf die Kontrollgewindespindel (610) einwirkt,
- einen Kontaktsensor (710, 711, 712, 713), um zu erfassen, ob Kontakt zwischen dem Schieber (300) und dem Spritzenkopf (401) besteht oder nicht.

8. Pumpe nach dem vorhergehenden Anspruch, **dadurch gekennzeichnet, dass** die Bremse (620) aus einer elektromagnetischen Bremse gebildet ist, die einen Rotor (625, 626, 627), der mit der Kontrollgewindespindel (610) verbunden ist, und einen Stator (621), der mit dem Gehäuse (100) verbunden ist, umfasst.

9. Pumpe nach Anspruch 7 oder 8, **dadurch gekennzeichnet, dass** der Schieber (300) mit dem Kontaktsensor (710, 711, 712, 713) versehen ist.

10. Pumpe nach einem der Ansprüche 7 bis 9, **dadurch gekennzeichnet, dass** die Antriebsmittel (500) aus einem Motor (502) und einer Antriebsgewindespindel (501), die durch den Motor (502) drehend angetrieben wird, gebildet sind.

11. Pumpe nach dem vorhergehenden Anspruch, **dadurch gekennzeichnet, dass** die Kupplungsmittel aus Folgendem gebildet sind: einem Entkupplungshebel (310), der sich außen am Gehäuse (100) befindet, einer mit dem Hebel (310) verbundenen Schieberwelle (311) und zwei Halbmuttern (351), die zwischen einer Kupplungsposition, in der sie die Antriebsgewindespindel (501) umgeben, sodass sie mit dieser zusammenwirken können, und einer Entkupplungsposition, in der sie von der Antriebsgewindespindel (501) beabstandet sind, sodass sie mit dieser nicht zusammenwirken, verschoben werden können.

12. Pumpe nach einem der Ansprüche 7 bis 11, **dadurch gekennzeichnet, dass** mindestens ein Kupplungssensor (720, 721, 722, 730) vorgesehen ist, um zu bestimmen, ob der Schieber (300) mit den Kupplungsmitteln gekuppelt oder von ihnen entkuppelt ist.

13. Pumpe nach dem vorhergehenden Anspruch, **dadurch gekennzeichnet, dass** der oder die Kupplungssensoren (720, 721, 722, 730) an dem Schieber (300) und/oder an den Kupplungsmitteln (350) angeordnet sind.

## Claims

1. Method of controlling the displacement of the push device of a pump of the syringe driver-type, the pump comprising a casing (100), a syringe cradle (200) in which a syringe (400) is placed, a push device (300) mobile relative to the casing (100) and capable of being driven in translation parallel to the longitudinal axis of the syringe by driving means (500), and clutch means (310, 311, 350, 351, 352) for engaging or disengaging the push device (300) on the driving means (500), method **characterized by** the following steps:
a) disengaging the push device (300) from the driving means (500);
b) detecting the presence or absence of contact between the push device (300) and the syringe head (401) by means of a contact sensor (710, 711, 712, 713);
c) moving the push device (300) in translation toward the syringe (400) if there is no contact between the push device and the syringe head, detecting the contact of the push device with the syringe head by means of the contact sensor and immediately actuating means for blocking the translation movement of the push device which had been inactivated up to then.

2. Method according to the preceding claim, **characterized by** the following additional steps performed after step c):
d) engaging the push device (300) on the driving means (500);
e) unblocking the translation movement of the push device (300).

3. Method according to the preceding claim, **characterized by** the following additional steps performed after step e):
f) disengaging the push device (300) from the driving means (500);
g) moving in translation, by the desired amplitude, the push device (300) in contact with the syringe head (401) in direction of the syringe (400);
h) stopping the movement of the push device (300) and engaging the push device on the driving means (500).

4. Method according to claim 1, **characterized by** the following additional steps performed after step b) in the case of detecting the presence of a contact between the push device (300) and the syringe head (401) at said step b):
g') moving in translation, by the desired amplitude, the push device (300) in contact with the syringe head (401) in direction of the syringe;
h') stopping the movement of the push device and engaging the push device on the driving means (500).

5. Method according to one of claims 2 to 4, **characterized by** the following additional step performed after step e) or h), respectively:
i) actuating the driving means (500) of the push device (300).

6. Method according to one of claims 2 to 4, said method being applied to a pump whose push device (300) is driven by a threaded driving rod (501) by means of two half-nuts (351) capable of being moved between an engaged position in which the two half-nuts (351) are brought close to each other while surrounding the threaded driving rod (501) so that they can move on it when it is set in rotation, and a disengaged position in which the two half-nuts (351) are brought away from each other and from the threaded driving rod (501) so that they no longer cooperate with it, **characterized by** the following intermediate steps performed after the engagement of steps d) or h), respectively:
d'/h") detecting the relative position of the half-nuts (351);
d"/h") if the two half-nuts (351) have not reached the engaged position, setting the threaded driving rod (501) in rotation, preferably by controlling the effort generated by the push device on the syringe via a force sensor integrated with the push device, until the two half-nuts (351) have reached the engaged position.

7. Pump of the syringe driver-type comprising a casing (100), a syringe cradle (200) intended to receive a syringe (400), a push device (300) mobile relative to the casing (100) and capable of being driven in translation parallel to the longitudinal axis of the syringe (400) by driving means (500), clutch means (310, 311, 350, 351, 352) for engaging or disengaging the push device (300) on the driving means (500), **characterized in that** the pump is equipped with means for blocking the movement of the push device (300) toward the syringe (400) when the push device contacts the syringe head (401) during a movement from a position without contact with the syringe head (401) toward a position in contact with the syringe head (401), the means for blocking the push device comprising
- a threaded control rod (610),
- a nut (611) engaged on the threaded control rod (610) and integral with the push device (300),
- a brake (620) acting on the threaded control rod (610),
- a contact sensor (710, 711, 712, 713) to detect the presence or the absence of contact between the push device (300) and the syringe head (401).

8. Pump according to the preceding claim, **characterized in that** the brake (620) is constituted by an electromagnetic brake comprising a rotor (625, 626, 627) integral with the threaded control rod (610) and a stator (621) integral with the casing (100).

9. Pump according to claim 7 or 8, **characterized in that** the push device (300) is equipped with the contact sensor (710, 711, 712, 713).

10. Pump according to one of claims 7 to 9, **characterized in that** the driving means (500) are constituted by a motor (502) and a threaded driving rod (501) driven in rotation by the motor (502).

11. Pump according to the preceding claim, **characterized in that** the clutch means are constituted by a clutch lever (310) located outside the casing (100), a push rod (311) integral with the lever (310) and two half-nuts (351) capable of being moved from an engaged position in which they surround the threaded driving rod (501) so as to be capable of cooperating with it and a disengaged position in which they are moved away from the threaded driving rod (501) so that they do not cooperate with it.

12. Pump according to one of claims 7 to 11, **characterized in that** at least one engagement sensor (720, 721, 722, 730) is provided to determine if the push device (300) is engaged or disengaged on the clutch means.

13. Pump according to the preceding claim, **characterized in that** the engagement sensor or sensors (720, 721, 722, 730) are placed on the push device (300) and/or on the clutch means (350).
